(19) Europäisches Patentamt
European Patent Office
Office européen des brevets

(11) Publication number: **0 286 887 B1**

(12) **EUROPEAN PATENT SPECIFICATION**

(45) Date of publication of patent specification: **11.11.92**

(51) Int. Cl.5: **C07D 211/90**, C08K 5/34, C08G 63/68, C08L 27/06

(21) Application number: **88104801.1**

(22) Date of filing: **25.03.88**

(54) **Poly 1,4-dihydro-2,6-dimethylpyridine-3,5-dicarboxylesters useful as thermal stabilizers for synthetic polymers.**

(30) Priority: **15.04.87 IT 201887**

(43) Date of publication of application:
**19.10.88 Bulletin 88/42**

(45) Publication of the grant of the patent:
**11.11.92 Bulletin 92/46**

(84) Designated Contracting States:
**AT BE CH DE ES FR GB GR IT LI LU NL SE**

(56) References cited:
**EP-A- 0 002 007**
**EP-A- 0 051 334**
**FR-A- 2 437 422**

(73) Proprietor: **LAGOR SPA**
**Via S. Giovanna d'Arco 7**
**Milano(IT)**

(72) Inventor: **Li Bassi, Giuseppe**
**Via Stretti 4**
**I-21026 Gavirate (VA)(IT)**
Inventor: **Ortica, Roberto**
**via N. Sauro 57**
**I-20025 Legnano (MI)(IT)**

(74) Representative: **Gervasi, Gemma, Dr.**
**NOTARBARTOLO & GERVASI Srl 33, Viale Bianca Maria**
**I-20122 Milano(IT)**

**EP 0 286 887 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to poly 1,4-dihydro-2,6-dimethylpyridine-3,5-dicarboxylesters of general formula (I)

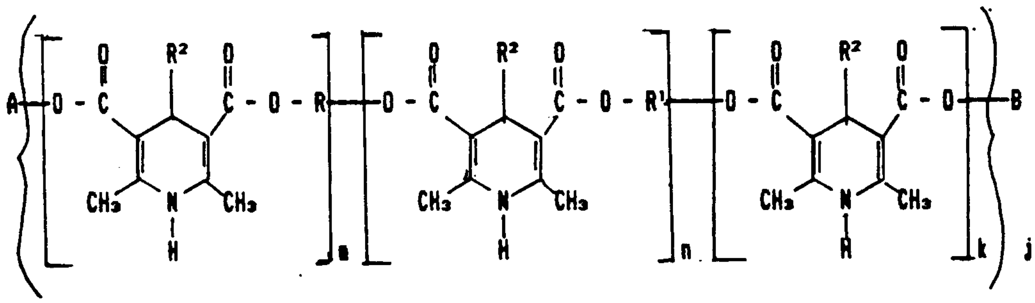

in which
A represents a linear or branched alkyl of 1-22 C atoms, possibly substituted with one or more groups chosen from alkoxy, alkylthio, hydroxyl possibly esterified with acrylic or methacrylic acid, halogen, aryl; phenyl or aryl of carbocyclic or heterocyclic type possibly substituted with one or more alkyl, alkoxy or halogen groups; alkenyl of 3-10 C atoms; $CH_3COCH_2$-COO-R-; $CH_3COCH_2COO$-$R^1$; $CH_3$-C($NH_2$) = CH-COOR-; $CH_3$-C($NH_2$) = CHCOO-$R^1$-; in which the amino group can carry one or more substituents of alkyl, hydroxyalkyl or alkoxyalkyl type or a cyclic substituent of polyalkylene or oxapolyalkylene type, or methylene or a linear or branched alkenyl of 2-22 C atoms;
B can assume the same meaning as A, or can represent a trivalent or polyvalent residue consisting of a linear or branched carbon atom chain possibly carrying substitutions of alkoxy, thioalkoxy, aryl, carboxyl or hydroxyl type;
m, n are whole numbers from 0 to 20, the sum of which is other than 0;
k can be 0 or 1;
j is a whole number from 1 to 6;
j (k + m + n) is a whole number greater than 1;
R and $R^1$ each independently represent a methylene or phenylene group, or one alkylene group of the type:

$$--(--C_pH_{2p} --- X ---)_t--- C_pH_{2p} --$$

in which p is a whole number from 2 to 18, t is a whole number from 0 to 10 and X can be oxygen or sulphur, said alkylene group possibly carrying substitutions of alkoxy or thioalkoxy, aryl, carboxyl or hydroxyl type; or R and $R^1$ represent a direct bond with B only if k is 0 and j is greater than 1;
$R^2$ represents hydrogen or a monovalent residue of linear or branched alkyl, alkoxycarbonyl or aryl type possibly substituted with one or more alkyl, alkoxy, halogen or $NO_2$ groups.

The sequence of units which are repeated m and n times (for m + n greater than or equal to 3) can be of alternating type (a), block type (b) or random type (c).

The compounds of general formula (I) are useful as thermal stabilizers for synthetic polymers, in particular for polyvinylchloride-based masses.

For the correct conversion of PVC-based masses by thermal processes and the subsequent preservation of the manufactured articles under photooxidative conditions, it is necessary to add to said masses primary thermostabilizers and photostabilizers which are mostly based on organic metal derivatives (Chevassus et al., The Stabilization of Polyvinylchloride, 1963). This practice has lead to the identification of precise combinations of said organic metal derivatives with other organic substances such as alkyl, aryl or arylalkyl phosphites, epoxy or phenol derivatives, possessing high stabilizing efficiency.

In these mixtures each component performs a specific role by means of curative action, ie action directed towards repairing any damage produced in the polymer matrix (modification by interruption of the conjugation in the polyene chain, or modification of its ionic state), or preventive action, ie action directed towards preventing possible molecular damage (hydrochloric acid capture, interruption of the chain dehydrohalogenation, primary radical capture or hydroperoxide decomposition, etc.) in the chain degradation process which is triggered by thermal or photochemical effect (R. Gaechter, H. Mueller, Taschenbuch der Kunststoff-Additive, C. Hanser Verlag, 2 Ausgabe, 1983).

Lead, cadmium and tin-based compounds have for many years been the mainstays of this stabilization

technique, and in part continue to be so. Given the general tendency towards the elimination of possible sources of toxicity in the environment, and because of the widespread distribution of PVC articles (10 million tons sold worldwide in 1980) the resources directed towards the use of primary stabilizer combinations which are progressively safer from the environmental toxicological viewpoint have been enormous. Although barium-zinc combinations have largely replaced barium-cadmium combinations, the modern tendency is towards calcium-zinc combinations which are so toxicologically safe as to be accepted by nearly all the legislation of industrialised countries, even in the case of articles intended for contact with food.

Unfortunately, calcium and zinc derivatives do not possess high thermal stabilization efficiency and require the co-use of specific compounds which not only give improved thermal stability but in addition do not prejudice photochemical stability. Thus use is now made of Ca/Zn carboxylate - epoxidised soya oil - organotin mercaptide and maleate combinations.

Prefecting these associations for industrial purposes has nevertheless required the co-use of organic co-stabilizers possessing high efficiency and low toxicity.

Compounds which have been studied and developed include alphaphenylindole (DE-PS 862512 - 1942 - Bayer AG), diphenylthiourea (DE-PS 746081 - 1940 - I.G. Farben Ind. A.G.), betaaminocrotonates [A. Michel, T.V. Hoang, A. Guyot, J. Macromol, Sci. Chem. A 12,411 (1978)], lactones containing carbonyl groups such as dehydroacetic acid (Belg. Pat. 875940 - 1979 - S.A. Argus) and more recently betadiketone derivatives such as dibenzoylmethane and stearoylbenzoylmethane (Fr. 2297227 and Fr. 23246871 - 1975 - Rhone Poulenc and JK 76, 111252 - 1975 - Akishima).

1,4-dihydro-2,6-dimethylpyridine-3,5-dicarboxylesters, originally proposed in 1973 as thermal stabilizers for PVC (FR 2239496 - 1973 - Societé Chimique des Charbonnages), have been more recently developed as thermal and photochemical stabilizers for PVC (FR 2405974, 2429806, 2433034 - 11977 - LABAZ), including in synergic combinations with 2-phenylindole derivatives (FR 2407236, 2439215 - 1977 - LABAZ), with organotin compounds (FR 79/25414 - 1979 - Omnium Financier Aquitaine pour l'Hygiene et la Sante), with betaaminocrotonates (FR 2491480 - 1980 - SANOFI), with betadiketones (FR 25344263 - 1982, EP 5678 - 1978, Rhone Poulenc; EP 51334 - 1981, Solvay), with phenolic and sulphurated antioxidants (EP 24754 - 1797 - Solvay), with zinc mercaptoester salts (US Appl. 269085 - 1981 - CARSTAB) and with various organometallic compounds (EP 2007 - 1979 - CIBA GEIGY).

However, some of these compounds have intrinsic drawbacks. For example when incorporated in PVC articles, betaaminocrotonates release ammonia with time or during thermal processing. Some dihydropyridines and alphaphenylindole easily sublime under PVC working conditions; some compounds such as dehydroacetic acid are unstable under heat and acidity; alphaphenylindole has poor light stability; other compounds are difficult to prepare; still others easily migrate or are extracted by the solutions which come into contact with articles prepared from them when used as wrapping for example for food.

It has now been surprisingly found that many of the aforesaid drawbacks do not occur, and that improved thermal stabilization of PVC-based masses containing primary stabilizers is obtained if the compounds according to the present invention are used in them, these being new compounds and being characterised by the molecular structure of formula (I) as defined heretofore.

Preferred compounds of formula (I) are those in which :

A, B which can be the same or different, each independently represent a linear or branched $C_1$-$C_{18}$ alkyl group;

m, k, j = 1

n = 0

R represents a $-(CH_2)_2-$, $-(CH_2)_4-$ or $-(CH_2)_2-S-(CH_2)_2-$ group;

$R^2$ represents hydrogen.

Examples of compounds represented by formula (I) are oligomers, polymers or in any event molecules of high molecular weight, characterised by containing two or more nuclei of 1,4-dihydro-2,6-dime thylpyridine-3,5-dicarboxylic acid (DHPC) variously esterified with monofunctional, difunctional or polyfunctional alcohols.

If the compounds of formula (I) are linear oligomers or polymers, A and B can represent residues of monovalent alcohols such as methyl, ethyl or dodecyl, whereas R and $R^1$ represent residues of bivalent alcohols such as ethylene or butylene glycol, thiodiglycol etc; in the case of polymers or oligomers deriving from different monomer units (R different from $R^1$) the sequence can be random, alternating or of block type.

When the compounds of formula (I) are branched polydihydropyridines, the heterocyclic units are not all connected in linear sequence and then for example A represents a residue of a monovalent alcohol such as methanol, ethanol etc., B represents a residue of a tri or polyvalent alcohol such as glycerin, trimethylol

propane, pentaerythritol, sorbitol etc., and R and $R^1$ have the same meaning as for linear polymers.

$R^2$ is preferably a linear or branched alkyl of methyl or isopropyl type, a phenyl or an alkoxycarbonyl.

Table 1 shows some combinations of chemical groupings which when inserted into formula (I) form structures which represent and exemplify the compounds according to the present invention.

TABLE 1

| SYMBOL | No. | A | B | R |
|---|---|---|---|---|
| B6E | 1 | $C_2H_5$ | A | $-(CH_2)_4-$ |
| | 2 | $CH_2=CH-COO(CH_2)_4-$ | A | $-(CH_2)_4-$ |
| T6D | 3 | $C_{12}H_{25}$ | A | $-(CH_2)_2-S-(CH_2)_2-$ |
| | 4 | CH3 | A | $-(CH_2)_4-$ |
| B63D | 5 | $C_{12}H_{25}$ | A | $-(CH_2)_4-$ |
| T2B1E | 6 | $C_2H_5$ | A | $-(CH_2)_2-S-(CH_2)_2-$ |
| | 7 | $C_{12}H_{25}$ | A | $-(CH_2)_4-$ |
| | 8 | $CH_3$ | A | $-(CH_2)_4-$ |
| | 9 | $C_6H_5$ | A | $-(CH_2)_4-$ |
| B6nAA | 10 | $CH_3COCH_2COO(CH_2)_4-$ | A | $-(CH_2)_4-$ |
| | 11 | $CH_3C(NH_2)=CHCOO(CH_2)_4-$ | A | $-(CH_2)_2-S-(CH_2)_2-$ |
| | 15 | $CH_3$ | A | $-(CH_2)_4-$ |
| | 16 | $CH_3$ | A | $-(CH_2)_2-S-(CH_2)_2-$ |
| T6nAC | 17 | $CH_3C(NH_2)=CHCOO-(CH_2)_2-S-(CH_2)_2-$ | A | $-(CH_2)_2-S-(CH_2)_2-$ |
| B6D | 18 | $C_{12}H_{25}$ | A | $-(CH_2)_4-$ |
| B63E | 19 | $C_2H_5$ | A | $-(CH_2)_4-$ |
| B67E | 20 | $C_2H_5$ | A | $-(CH_2)_4-$ |
| B6M | 21 | $CH_3$ | A | $-(CH_2)_4-$ |
| T6E | 22 | $C_2H_5$ | A | $-(CH_2)_2-S-(CH_2)_2-$ |
| T6M | 23 | $CH_3$ | A | $-(CH_2)_2-S-(CH_2)_2-$ |
| T4B3E | 24 | $C_2H_5$ | A | $-(CH_2)_2-S-(CH_2)_2-$ |

TABLE 1 (continued)

| Symbol | No. | R' | R² | m | n | k | i | sequence |
|---|---|---|---|---|---|---|---|---|
| BGE | 1 | - | H | 1 | 0 | 1 | 1 | - |
| | 2 | - | H | 1 | 0 | 1 | 1 | - |
| TGD | 3 | - | H | 1 | 0 | 1 | 1 | - |
| | 4 | $-(CH_2)_2-S-(CH_2)_2-$ | H | 1 | 1 | 1 | 1 | - |
| BG3D | 5 | - | H | 3 | - | 1 | 1 | - |
| T2B1E | 6 | $-(CH_2)_4-$ | H | 2 | 1 | 1 | 1 | a |
| | 7 | $-(CH_2)_2-S-(CH_2)_2-$ | H | 3 | 2 | 1 | 1 | a |
| | 8 | $-(CH_2)_2-S-(CH_2)_2-$ | H | 3 | 4 | 1 | 1 | b |
| | 9 | $-(CH_2)_2-O-(CH_2)_2-$ | H | 12 | 6 | 1 | 1 | c |
| BGnAA | 10 | - | H | 15 | - | 1 | 1 | - |
| | 11 | $-(CH_2)_4-$ | H | 4 | 4 | 1 | 1 | a |
| | 12 | - | H | 3 | 0 | 0 | 1 | - |
| | 13 | - | H | 4 | 0 | 0 | 1 | - |
| | 14 | $-CH_2-$ | H | 1 | 1 | 0 | 1 | - |
| | 15 | - | $CH_3$ | 1 | 0 | 1 | 1 | - |
| | 16 | - | $C_6H_5$ | 1 | 0 | 1 | 1 | - |
| TGnAC | 17 | - | H | 15 | - | 1 | 1 | - |
| BGD | 18 | - | H | 1 | 0 | 1 | 1 | - |
| BG3E | 19 | - | H | 3 | 0 | 1 | 1 | - |
| BG7E | 20 | - | H | 7 | 0 | 1 | 1 | - |
| BGM | 21 | - | H | 1 | 0 | 1 | 1 | - |
| TGE | 22 | - | H | 1 | 0 | 1 | 1 | - |
| TGM | 23 | - | H | 1 | 0 | 1 | 1 | - |
| T4B3E | 24 | $-(CH_2)_4-$ | H | 4 | 3 | 1 | 1 | - |

It has also been surprisingly found that in PVC-based masses containing primary stabilizers the combined use of compounds of formula (I) together with betadiketone compounds of formula (II) or together with metallic or organotin salts of enolates of compounds of formula (II):

$R^3-CO-CH_2-CO-R^4$ (II)

in which:
$R^3$ represents linear or branched $C_1$-$C_{18}$ alkyl, or an aromatic phenyl ring possibly substituted with one or more alkyl, alkoxy or halogen groups;
$R^4$ has the same meaning as $R^3$, independently of $R^3$, or represents a covalent bond, $C_1$-$C_{18}$ alkylene, polyvalent $C_1$-$C_{18}$ hydrocarbon, or $-(C_pH_{2p}-X)_t-C_pH_{2p}-$ in which p, t, X have the meanings already given for formula (I), and directly linked to another $-CO-CH_2-CO-R^3$ group,
results in thermal stabilization of synergic type exceeding the sum of that of the individual components namely compounds (I) and compounds (II).

Compounds of formula (II) are known in the PVC stabilization field; examples are benzoylstearoylmethane, dibenzoylmethane (FR 2297227 and Fr 2324681), 1,12-dibenzoyldodecane-2,11-dione and 1,4-dibenzoylbutane-2,3-dione (EP 14508 and EP 46161).

Compounds of formula (I) according to the present invention are obtained by suitable use of Hantzsch synthesis in one of the three most known variations [U. Eisner, J. Kuthan, Chem. Rev. 72 (1), 1-42 (1972); F. Bossert, H. Meyer, E. Wehiger, Angew. Chem. Int. Ed. Eng., 20, 762-765 (1981)], as follows:

A) Condensation of an aldehyde with a bisacetonacetate and ammonia with water elimination [the corresponding esters of betaaminocrotonic acid of formula (IV) are obtained as intermediates]

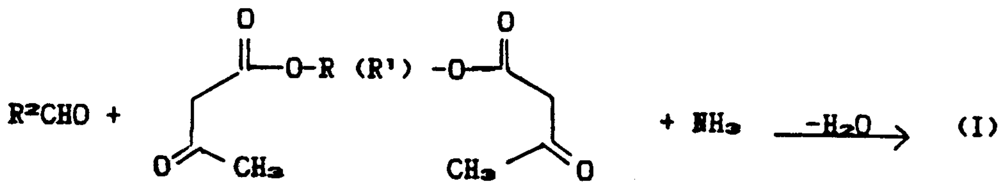


(III) (IV)

j(m+n+k) mol j(m+n) mol j(m+n+k) mol

By using mixtures of acetoacetates (IV) (R different from R[1]), compounds of mixed composition and random sequence are obtained.

The molecular weight and thus the length of the linear oligomer or polymer chain can be regulated by introducing an adequate predetermined quantity of monofunctional acetoacetates (V) or betaaminocrotonates (VII) which act as chain terminators:

$CH_3$-CO-$CH_2$-COO-X (V)

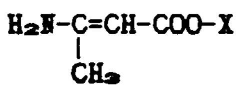
(VII)

in which in the monofunctional compound X = $CH_3$, $C_2H_5$ or $C_{12}H_{25}$; in the tri or polyfunctional compound X = $C(CH_2-)_4$ or $CH_3-CH_2-C(CH_2-)_3$.

If non-linear oligomers are to be obtained, the terminators used must be monofunctional acetoacetates (V), (j moles) together with tri or polyfunctional acetoacetates (containing j functions) (1 mole), such as pentaerythrityl tetrakisacetoacetate.

The ammonia can be introduced in the form of gas or is preferably generated in situ from labile compounds such as ammonium carbonate or ammonium acetate, or from hexamethylenetetramine if the aldehyde is formaldehyde.

B) Condensation of an aldehyde with a bis-betaaminocrotonate and elimination of water and ammonia:

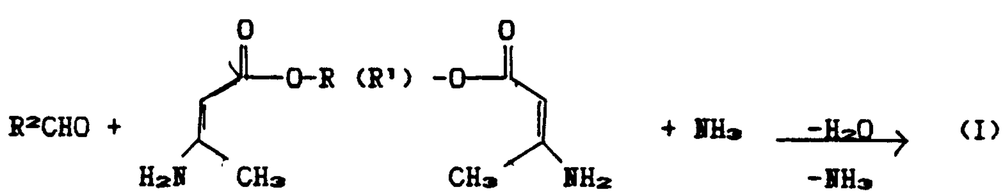


(III) (VI)

j(m+n+k) mol j(m+n) mol

By using mixtures of betaaminocrotonates (VI) (R different from R[1]), mixed compounds of random

sequence are obtained. The molecular weight and thus the length of the linear oligomer or polymer chain can be regulated by introducing an adequate predetermined quantity of monofunctional betaaminocrotonates (VII) or acetoacetate (V) to act as chain terminators.

If non-linear oligomers are required, the chain terminators used must be betaaminocrotonates (VII) (j moles) together with betaaminocrotonates of tri or polyfunctional type (containing j amino groups) (1 mole) such as trimethylolpropane trisbetaaminocrotonate.

C) Condensation of an aldehyde with a bis-betaaminocrotonate and a bis-acetoacetate and elimination of water:

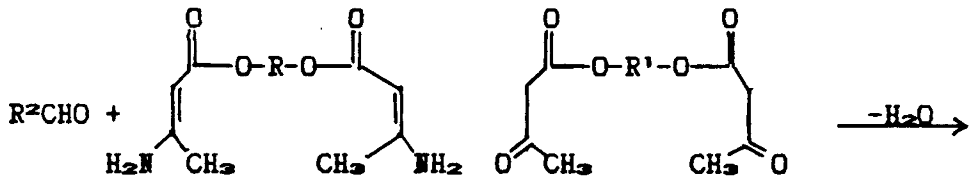


(I)

(III) (VI) (IV)

j(m+n+k) mol (jm) mol (jn) mol

By using the synthetic scheme in sequential controlled manner, ie implementing the condensation in consecutive stages with different ratio pairs of acetoacetates and betaaminocrotonates, it is possible to obtain oligomers or polymers of alternating sequence or block type in accordance with a predetermined structure. The molecular weight and thus the oligomer or polymer chain length is regulated by introducing suitable predetermined quantities of monofunctional terminators of the aminocrotonate type (VII) or acetoacetate type (V).

In the particular case of dimer compounds, the following synthesis schemes are used:

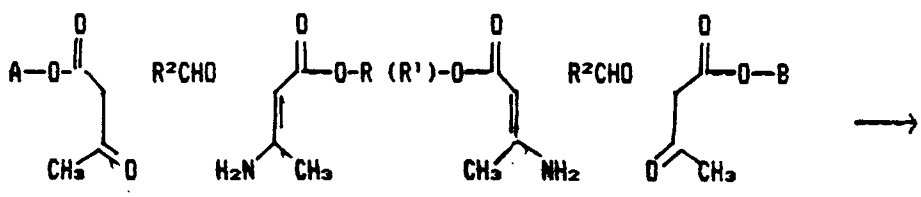

(VIII)

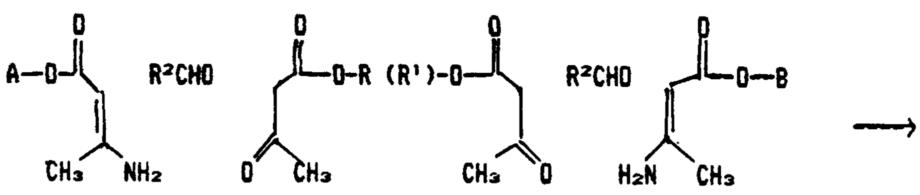

(VIII)

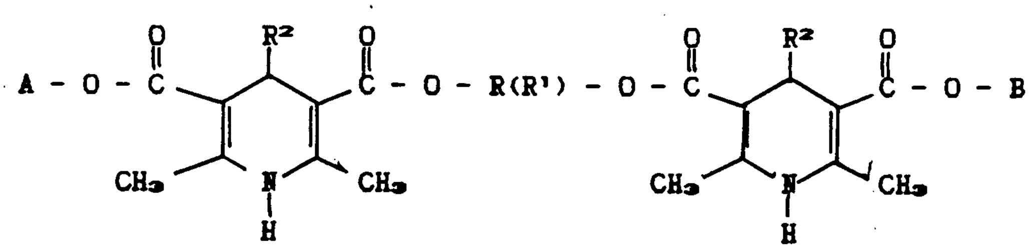

(VIII)

If oligomers, polymers or compounds of branched type are to be obtained, the chain terminators used must be monofunctional acetoacetates (V) or betaaminocrotonates (VII) (j moles) together with tri or

polyfunctional acetoacetates (V) or betaaminocrotonates (VII) (containing j functions) (1 mole).

This method is the most versatile and is preferred for preparing compounds of formula (I).

The standard reaction conditions for schemes A, B and C comprise the use of a low-boiling alcoholic solvent, preferably isopropyl alcohol, and a heating stage of 1-20 hours. During the reaction the reagents, initially not all soluble, pass into solution and on termination generally a crystalline precipitate forms. The solvent is then removed or the reaction mixture diluted with water. After separation by filtration, the reaction product can be purified by crystallisation or by repeated washing with a suitable solvent.

The compounds of formula (I) are generally crystalline solids of defined melting point, light yellow in colour and possessing fluorescence, they being poorly soluble in common organic solvents and practically insoluble in water.

The following examples illustrate the preparation of compounds of formula (I) and their chemico-physical characteristics. Parts and percentages are by weight.

EXAMPLE 1 (Synthesis from acetoacetates):

Poly [1,4-dihydro-2,6-dimethyl-3,5-dicarboxypyridine-monobutylene ester] (BGnAA, compound 10, Table 1)

51.65 g of dibutyleneglycolbisacetoacetate, 3.1 g of methylacetoacetate, 10.5 g of hexamethylenetetramine, 2.9 g of ammonium acetate of 60 ml of isopropylalcohol containing 20 g of water are placed in a 250 ml flask. The mixture is heated to 60 ° C over 1 hour and kept at this temperature for 1 hour, after which it is heated under reflux for 3 hours and then left at rest overnight at 20 ° C. The suspension is poured into water under effective agitation. The yellow solid obtained is separated by filtration and washed repeatedly with water and then with acetone. After drying at 40 ° C under vacuum, 42.7 g of product are obtained with a M.P. of 175 ° C.

EXAMPLE 2 (Synthesis from betaaminocrotonates)

Poly [1,4-dihydro-2,6-dimethyl-3,5-dicarboxypyridinemonothiodiglycolester] (TGnAC, compound 17, Table 1)

8.65 g of thioglycolbisaminocrotonate, 0.49 g of methylbetaaminocrotonate, 3.0 g of aqueous formaldehyde(38% by weight) and 50 ml of isopropylalcohol are placed in a 250 ml flask. The mixture is heated to 60 ° C over 1 hour and then under reflux for 6 hours, after which it is left at rest overnight at 20 ° C. The suspension is poured into water under effective agitation. The yellow solid obtained is separated by filtration, washed repeatedly with water and then with acetone. After drying at 40 ° C under vacuum, 7.38 g of product are obtained with a M.P. of 156 ° C.

EXAMPLE 3 (Mixed synthesis)

(TGD, compound 3, Table 1)

150 ml of isopropylalcohol, 14.6 g of thiodiglycolbisaminocrotonate, 28.2 g of dodecylacetoacetate and 3 g of paraformaldehyde are placed in a 250 ml flask. The mixture is agitated under nitrogen for 10 minutes and then heated under reflux for 40 minutes. 80 ml of water are added and heating continued at 80 ° C for 30 minutes. The mixture is cooled to 20 ° C, the yellow solid obtained is filtered off and washed with water and then with 80 ml of acetone. It is dried under vacuum at 40 ° C to obtain 32.5 g of product of M.P. 146 ° C.

EXAMPLE 4 (Mixed synthesis)

(BG3D, compound 5, Table 1)

100 ml of isopropylalcohol, 25.6 g of butyleneglycolbisaminocrotonate, 12.9 g of butyleneglycolbisacetoacetate and 6 g of paraformaldehyde are placed in a 250 ml flask at 20 ° C. The mixture is agitated at 20 ° C for 10 minutes and then at 80 ° C for 120 minutes. 27.04 g of dodecylacetoacetate dissolved in 30 ml of isopropylalcohol are then fed in over 10 minutes.

The mixture is kept at 80 ° C for 1 hour after which 80 ml of water are added.

After 30 minutes the mixture is cooled to 20°C and the solid which forms is filtered off, washed with water and then with 50 ml of acetone. It is dried under vacuum at 40°C to obtain 54 g of product of M.P. 168°C.

Other compounds were obtained in an analogous manner, their chemico-physical and chemical characteristics being shown in Tables 2 and 3.

Spectroscopic characteristics (infrared, ultraviolet and NMR spectra), elementary analysis, thin layer chromatographic data and the synthesis procedure confirm the structure of the products obtained.

TABLE 2

(General physico-chemical characteristics)

| Comp. symbol | BGM | BGE | BG3E | BG7E |
|---|---|---|---|---|
| No. (Tab. 1) | 21 | 1 | 19 | 20 |
| APPEARANCE | fluorescent light yellow powder | | | |
| MELTING POINT °C | 183 | 168 | 173 | 154 |
| INFRARED SPECTRUM (Absorption, $cm^{-1}$) | 3355<br>1700<br>1660<br>1510<br>1305<br>1220<br>1115<br>750 | 3360<br>1700<br>1650<br>1510<br>1305<br>1220<br>1120<br>745 | 3355<br>1700<br>1660<br>1510<br>1305<br>1220<br>1115<br>750 | 3355<br>1700<br>1650<br>1505<br>1220<br>1105<br>750 |
| TLC (silica, eluent chloroform 4, acetone 1) ($R_f$) | 0.50 | 0.47 | 0.02 | 0.01 |
| SOLUBILITY (g/l) | | | | |
| Water | insoluble | insoluble | insoluble | insoluble |
| Gl. acetic acid | < 0.25 | | | |
| 95% ethanol | < 0.3 | < 0.3 | | |
| Chloroform | 0.5 | | | |
| Acetone | 0.6 | | | |
| Dioctylphthalate | < 0.2 | | | |
| VOLATILITY % weight loss at 210°C for 10 minutes | 6 | | | |
| EMPIRICAL FORMULA | $C_{24}H_{32}ON_2O_8$ | $C_{26}H_{36}N_2O_8$ | $C_{52}H_{70}N_4O_{16}$ | $C_{104}H_{138}N_8O_{32}$ |
| MOLECULAR WEIGHT | 476.35 | 504.57 | 1007.14 | 2012.3 |

TABLE 2 (continued)

(General physico-chemical characteristics)

| Comp. symbol | T6E | T6D | T2B1E | T4B3E | T6nAC |
|---|---|---|---|---|---|
| No. (Tab. 1) | 22 | 3 | 6 | 24 | 17 |
| APPEARANCE | fluorescent light yellow powder | | | | |
| MELTING POINT °C | 146 | 146 | 125 | 127 | 156 |
| INFRARED SPECTRUM | 3350 | 3345 | 3350 | 3350 | 3350 |
| (Absorption, $cm^{-1}$ | 1700 | 1700 | 1700 | 1700 | 1700 |
| | 1650 | 1660 | 1660 | 1660 | 1660 |
| | 1500 | 1500 | 1500 | 1500 | 1500 |
| | 1385 | 1296 | 1385 | 1385 | 1300 |
| | 1300 | 1220 | 1220 | 1210 | 1210 |
| | 1220 | 1120 | 1110 | 1110 | 1110 |
| | 1110 | 1100 | 1100 | 1010 | 1100 |
| | 1100 | 965 | 756 | 760 | 1005 |
| | 1010 | 750 | | | 750 |
| | 750 | | | | |
| TLC (silica, eluent chloroform 4, acetone 1) ($R_f$) | 0,46 | 0,67 | 0 | 0 | 0 |
| SOLUBILITY (g/l) | | | | | |
| Water | insoluble | insoluble | insoluble | insoluble | insoluble |
| Gl. acetic acid | | < 0,2 | | | |
| 95% ethanol | | < 0,2 | | | |
| Chloroform | | 20 | | | |
| Acetone | | < 0,15 | | | |
| Dioctylphthalate | | < 0,1 | | | |
| VOLATILITY % weight loss at 210°C for 10 minutes | | 1,3 | | | 1,5 |
| EMPIRICAL FORMULA | $C_{26}H_{36}N_2O_8S$ | $C_{46}H_{76}N_2O_8S$ | $C_{52}H_{70}N_4O_{18}S$ | $C_{104}H_{138}N_8O_{32}S_4$ | $C_{206}H_{270}N_{16}O_{64}S_{15}$ |
| MOLECULAR WEIGHT | 536,64 | 817,16 | 1071,26 | 2140,54 | 4474,37 |

TABLE 2

(General physico-chemical characteristics)

| Comp. symbol | BGD | BG3D | BGnAA | TGM |
|---|---|---|---|---|
| No. (Tab. 1) | 18 | 5 | 10 | 23 |
| APPEARANCE | fluorescent light yellow powder | | | |
| MELTING POINT °C | 165 | 168 | 175 | 157 |
| INFRARED SPECTRUM (Absorption, $cm^{-1}$ | 3360<br>1700<br>1660<br>1510<br>1220<br>750 | 3350<br>1695<br>1650<br>1505<br>1305<br>1220<br>750 | 3350<br>1695<br>1660<br>1505<br>1303<br>1220<br>750 | 3350<br>1697<br>1660<br>1505<br>1302<br>1225<br>1120<br>750 |
| TLC (silica, eluent chloroform 4, acetone 1) ($R_f$) | 0.69 | 0.05 | 0 | 0.42 |
| SOLUBILITY (g/l) | | | | |
| Water | insoluble | insoluble | insoluble | insoluble |
| Gl. acetic acid | < 0.2 | | < 0.2 | < 0.2 |
| 95% ethanol | < 0.2 | | | < 0.2 |
| Chloroform | 1.6 | | | 6.7 |
| Acetone | 1.2 | | 0.5 | < 0.35 |
| Dioctylphthalate | < 0.13 | | | < 0.1 |
| VOLATILITY % weight loss at 210°C for 10 minutes | 2.01 | | 1.6 | 7.8 |
| EMPIRICAL FORMULA | $C_{46}H_{76}N_2O_8$ | $C_{72}H_{110}N_4O_{16}$ | $C_{206}H_{270}N_{16}O_{64}$ | $C_{24}H_{32}N_2O_8S$ |
| MOLECULAR WEIGHT | 785.10 | 1287.68 | 3393.38 | 508.58 |

CHARACTERISATION EXAMPLES

BGD (compound 18)

- NMR spectrum ($CDCl_3$):

| δ (ppm) | 0,86 | 1,25 | 1,60 | 1,76 | 2,18 | 3,28 | 4,09 | 6,15 |
|---|---|---|---|---|---|---|---|---|
| multipl. | t | s | q | m | s | s | t | s |
| No. H | 6 | 36 | 4 | 4 | 12 | 4 | 8 | 2 |

- UV spectrum ($CHCl_3$) : λmax = 242 nm, $\epsilon_{242}$ = 17230 ($M^{-1}cm^{-1}$)
  λmax = 360 nm, $\epsilon_{360}$ = 12178 ($M^{-1}cm^{-1}$)

- Elementary analysis :

| | C | H | N |
|---|---|---|---|
| calculated | 70,37 | 9,75 | 3,57 |
| found | 69,60 | 9,82 | 3,38 |

TGM (compound 23)

- NMR spectrum ($P_yD_5$) :

| δ (ppm) | 2,48 | 3,09 | 3,81/3,82 | 4,6 | 9,5 |
|---|---|---|---|---|---|
| multipl. | 2s | t | 2s | t | s (broad) |
| No. H | 12 | 4 | 6 + 4 | 4 | 2 |

- UV spectrum ($CHCl_3$) : λmax = 244 nm, $\epsilon_{244}$ = 16419 ($M^{-1}cm^{-1}$)
  λmax = 364 nm, $\epsilon_{364}$ = 11411 ($M^{-1}cm^{-1}$)

- Elementary analysis :

| | C | H | N |
|---|---|---|---|
| calculated | 56,58 | 6,33 | 5,50 |
| found | 55,82 | 6,28 | 5,27 |

BGnAA (compound 10)

- NMR spectrum ($P_yD_5$) :

| δ (ppm) | 1,92 | 2,48 | 3,87 | 4,4 | 9,0 |
|---|---|---|---|---|---|
| multipl. | m | s | s | m | s (broad) |
| No. H | 4 | 6 | 2 | 4 | 1 |

- UV spectrum ($CHCl_3$) : λmax = 242 nm, $\epsilon_{242}$ = 74875 ($M^{-1}cm^{-1}$)
  λmax = 367 nm, $\epsilon_{367}$ = 37440 ($M^{-1}cm^{-1}$)

- Elementary analysis :

| | C | H | N |
|---|---|---|---|
| calculated | 61,95 | 6,81 | 5,6 |
| found | 61,17 | 6,89 | 4,49 |

TGD (compound 3)

- NMR spectrum ($CDCl_3$):

| δ (ppm) | 0,85 | 1,25 | 1,62 | 2,17/2,18 | 2,85 | 3,25 | 4,08 | 4,15 | 5,2 |
|---|---|---|---|---|---|---|---|---|---|
| multipl. | t | s | q | 2s | t | s | t | t | s |
| No. H | 6 | 36 | 4 | 12 | 4 | 4 | 4 | 4 | 2 |

- UV spectrum ($CHCl_3$) : $\lambda max$ = 245 nm, $\epsilon_{245}$ = 16397 ($M^{-1}cm^{-1}$)
  $\lambda max$ = 365 nm, $\epsilon_{365}$ = 12738 ($M^{-1}cm^{-1}$)

- Elementary analysis :

| | C | H | N |
|---|---|---|---|
| calculated | 67,09 | 9,37 | 3,42 |
| found | 66,78 | 9,43 | 3,33 |

The compounds of formula (I) according to the present invention are advantageously used in the thermal stabilization of synthetic polymers, particularly of masses based on PVC (obtained by polymerisation undiluted, in suspension or in emulsion), on various copolymers such as vinyl chloride/vinyl esters and on vinyl chloride/vinylidene chloride copolymers.

It has been surprisingly found that the use of said compounds in said masses in association with common primary Ca/Zn or Mg/Zn stabilizers, such as Ca/Zn stearates, when used mainly in association with epoxidised soya oil and organic mono or dioctyltin salts, in formulations suitable for the production of either rigid or flexible articles, results in high-efficiency thermal stabilization.

In particular, in a stabilizing mixture consisting of Ca/Zn stearates, epoxidised soya oil and dioctyltin bisisooctylthioglycolate, it is possible to partly or totally replace the tin derivative with a much smaller quantity of compounds of formula (I) either alone or in combination with compounds of formula (II) to obtain equal thermal stabilization efficiency.

The compounds of formula (I) either alone or in combination with compounds of formula (II) can also be used in PVC masses in association with primary stabilizers of Ba/Zn or Ba/Cd type. For example in the form of their maleic, 2-ethylhexanoic or 4-tertbutylbenzoic acid salts, commonly used together with organic phosphites such as tris-nonylphenylphosphite, decyldiphenylphosphite, tridecylphosphite etc., or can be used in association with stabilizers based on organic or inorganic lead derivatives, with advantages analogous to those stated heretofore.

The quantity of compounds of formula (I) to be used varies from 0.01 to 3% by weight of the polymer to be stabilized, and preferably between 0.1 and 0.5%.

It has also been surprisingly found that if, in masses containing primary stabilizers, compounds of formula (I) are used together with organic stabilizers such as betadicarbonyl compounds, a synergic stabilization effect is obtained, ie exceeding the sum of the effects of the compounds of formula (I) and the said stabilizers of formula (II) taken individually. Particularly preferred is the synergic combination with betadiketone compounds of formula (II) such as benzoylstearoylmethane, dibenzoylmethane, 1,4-dibenzoylbutane-2,3-dione, benzoylacetone, 1,12-dibenzoyldodecane-2,11-dione or metal or organotin salts of the respective enolates. The ratio of compounds of formula (I) to betadicarbonyl compounds of formula (II) can lie between 1:10 and 10:1, and preferably between 1:2 and 4:1.

A further aspect of using compounds of formula (I) derives from the fact that the masses obtained have excellent stability under photooxidative conditions when subjected to the action of light under natural conditions by exposure to solar light or to radiation of lamps of various kinds.

The stability is much better than in the case of masses stabilized with 2-phenylindole derivatives or betadiketones.

The stabilizers of formula (I) can be incorporated either alone or together with compounds of formula (II) into PVC-based masses by simply mixing the compounds together with all the required ingredients, using a slow mixer, turbo mixer, Banbury mixer, extruder or double-roller mixer to obtain maximum uniformity before thermal transformation to produce the finished article.

The stabilized masses containing compounds of formula (I) either alone or together with compounds of formula (II) can be used for transformation operations comprising a thermal cycle, such as calendering,

extrusion, injection-moulding, blow-moulding, thermoforming, spreading or rotary moulding, to obtain rigid or flexible articles, or expanded articles if suitable expanding agents are used.

The preferred uses of stabilized masses containing compounds of formula (I) possibly together with compounds of formula (II) are the manufacture of bottles by blow-moulding (blow-extrusion), rigid packages for food (for example by calendering), packaging in general by calendering and extrusion (possibly followed by vacuum-forming), film for general use, moulded or extruded articles etc.

Depending on the type of final use of the stabilizised masses, other additives can also be incorporated such as lubricants, mineral fillers, dyes, pigments, plasticisers, impact-strength modifiers, expanding agents, antiblocking agents, primary and secondary antioxidants, light stabilizers of various types such as UV radiation absorbers, optical bleaches etc.

The advantages consequent on the use of compounds of formula (I) either alone or in combination with compounds of formula (II) in PVC-based masses are various, and positively influence the transformation technology and the use of the final articles, and can be summarised as follows:

- a very high thermal stability (time necessary for carbonisation at a determined working temperature, under static or dynamic conditions);
- very high thermal stability of colour and transparency (time for which the colour and transparency of the article remain within a predetermined standard at a given working temperature);
- absence of influence on lubrication properties (ability to hot-work the mass without it adhering to the machine walls or undergoing plastic flow);
- absence of deposits on the machine due to the absence of sublimation when compounds of formula (I) are used either alone or with high molecular-weight compounds of formula (II);
- reduction in formulation costs because of the high stabilizing efficiency of compounds of formula (I) used either alone or together with compounds of formula (II) (possibility of reducing the quantities of base stabilizers or additives normally used);
- improvement in environmental safety during formulation and transformation, by allowing reduction in the quantity of compounds considered ecologically unsafe such as cadmium, lead or organotin derivatives, while maintaining equal stabilizing effect;
- high resistance to degradation by light under photooxidative conditions, even at high ambient temperature (50-70 °C) and in the presence of water;
- migration extremely reduced or absent by virtue of the poor solubility of compounds of formula (I) in nearly all cold substances due to their molecular weight, coupled with their affinity for PVC-based masses;
- reduction in toxicity of manufactured articles.

The following non-limiting examples illustrate in detail the applicational properties of compounds of formula (I) as thermal stabilizers for PVC-based masses.

Parts are by weight, and percentages refer to 100 parts of vinyl resin (PHR).

FORMULATIONS

1. Mixes for rigid transparent articles.

| | A | B | C | D |
|---|---|---|---|---|
| S-PVC (K58) (1) | 100 | 100 | 100 | 80 |
| PVA (2) | - | - | - | 20 |
| MBS (modifier) (3) | 8 | 6 | 6 | 8 |
| PMMA (working auxiliary) (4) | 1.5 | 1.5 | 1 | - |
| ESO (epoxidised soya oil) (5) | 2.5 | 2 | 2 | 2 |
| Cetylstearyl alcohol | 1 | 1 | 1.5 | 0.5 |
| Calcium stearate | 0.4 | 0.36 | 0.36 | 0.3 |
| Zinc stearate | 0.4 | 0.2 | variab. | 0.3 |
| Organic stabilizer | | | variable | |

(1) Sicron 230 (Montedison)

(2) Polyvinylacetate, Vinnol H 1157 (Wacker)

(3) Polymethylmethacrylate/butadiene/styrene, Paraloid 357 (Rohm & Hass)

(4) Polymethylmethacrylate, Paraloid K175 (Rohm & Hass)

(5) Edenol D 81 (Henkel)

2. Mixes for rigid pigmented articles.

| | |
|---|---|
| S-PVC (K 58) | 100 |
| PMMA | 1.5 |
| Cetylstearyl alcohol | 1 |
| Calcium carbonate (1) | 2 |
| Calcium stearate | 1 |
| Zinc stearate | 1 |
| Organic stabilizer | variable |

(1) BL R 3 (Omya)

3. Mixes for plasticised articles

| | A | B |
|---|---|---|
| S-PVC (K 65) (1) | 100 | - |

| | | |
|---|---|---|
| S-PVC (K 58) | - | 100 |
| DOP (dioctylphthalate) | 30 | 30 |
| ESO (epoxidised soya oil) | 3.0 | - |
| Calcium stearate | 0.4 | - |
| Zinc stearate | 0.4 | - |
| Pentaerythritol | 0.3 | - |
| Barium nonylphenolate carbonate (2) | - | 0.6 |
| Zinc 2-ethylhexanoate | - | 0.18 |
| Decyldiphenylphosphite | - | 0.4 |
| Organic stabiliser | | variable |

(1) Sicron 540 (Montedison)

(2) Lubrizol 2106 (Lubrizol)

TEST METHODS

1. Preparation of mixes

1A - For static thermostability:
The mixture of ingredients is gelled in a double-roller mixer at 175°C for 2 minutes (friction ratio 1:1). The sheet of 0.6 mm thickness is cooled rapidly on a flat surface.
1B - For dynamic thermostability:
The mixture of ingredients is gelled directly in a two-roller mixer at the temperature chosen for the stability test (friction ratio 1:1.5) which is then carried out. The sheet thickness is 0.6 mm.

2. Dynamic stability test

The mixture prepared as under point 1B is immediately passed to the two-roller mixer at the established temperature (friction ratio 1:1.5). At regular time intervals, sheet samples are withdrawn and are rapidly cooled on a flat surface. The test continues until incipient carbonisation. The time required to attain this is known as the long-term stability time. The stability values at intermediate times are evaluated by comparing the colour of the respective sheet samples, measured by the yellowing index (YI) in accordance with ASTM D 1925-70 standard. The longer the long-term stability time and the lower the yellowing index, the more thermally stable the material.

3. Static stability test

The sheet pregelled as under point 1A is cut into 3 x 3 cm pieces, which can be treated thermally in a temperature-controlled oven from which they are extracted at regular time intervals.

Alternatively, ageing can be effected in a temperature-controlled press (3 Atm) for different times.

The sheets, contained between mirror surfaces, measure 0.6 mm in thickness.

The long-term and intermediate stabilities are evaluated using the same criteria as for dynamic stability.

Both in the case of static stability and in the case of dynamic stability the yellowing indices are measured until incipient carbonisation (the time associated with the latter then being long-term stability).

4. Lubrication test

Lubrication is evaluated by carrying out the test in the same manner as described for dynamic stability, but measuring the total time which passes before the mixture sticks to the rollers.

The longer the measured time the more lubricated the material.

5. Transparency test

The sheet prepared as under point 1A is inserted into a press (3 Atm) between polished surfaces to obtain strips of 0.6 mm thickness. The coefficient of reflection on a black background and on a white background are measured through the depth of the strips obtained. Transparency, defined as the contrast ratio, is given by the ratio of the coefficient of reflection against the black background to that against the white background (ASTM D 28055-70).

The greater this ratio the poorer the transparency.

6. Plate out test

This test is carried out as for the dynamic stability test but interrupting mixing in the two-roller mixer before burning. The mixture used contains a red pigment.

Without cleaning the mixer rollers a white pigmented ($TiO_2/CaCO_3$) mixture is then used for 2 minutes. The colour assumed by this second mixture is evaluated by arbitrary units (0 to 10). The greater the colour the greater the plate out.

The following examples relate to the application of compounds of formula (I) in the aforestated formulations.

EXAMPLE 5

Dynamic stability was evaluated at 180 °C on the formulation for rigid transparent articles (1A) using 0.3 PHR of organic stabilizer. The YI values are given.

| Time (minutes) | 5 | 10 | 15 | 20 | 25 |
|---|---|---|---|---|---|
| without stabilizer | 37.8 | 47.5 | 52.7 | 54.7 | 75.5 |
| FI (1) (a) | 23.9 | 28 | 30.9 | 33.8 | 39.2 |
| BGAC (2) (a) | 17.0 | 19.4 | 30.2 | 72.8 | sticks |
| TGAC (3) (a) | 18.1 | 19.8 | 29.6 | 66 | sticks |
| DHPL (4) (a) | 17.2 | 18.2 | 21.2 | 31.2 | 58.9 |
| SBBM (5) (a) | 10.2 | 15.6 | 29.8 | 52.6 | 93.7 |
| BGD | 16.5 | 17.9 | 20.3 | 27.7 | 58.8 |
| TGD | 16.8 | 17 | 19 | 26.2 | 58 |
| TGM | 15.8 | 15.8 | 16.8 | 22.9 | 58.5 |
| BG3D | 16 | 17.5 | 19.5 | 26.5 | 58.8 |
| BGnAA | 16.2 | 17.8 | 19.6 | 26.7 | 58.6 |

(1) 2-phenylindole

(2) butylglycol bisbetaaminocrotonate

(3) thiodiglycol bisbetaaminocrotonate

(4) 1,4-dihydro-2,6-dimethyl-3,5-dicarboxypyridinedidodecyl ester

(5) sebacoyl bisbenzoylmethane

(a) used as reference

It will be noted that the compounds of the present invention are effective thermal stabilizers and substantially better than the compounds normally used. A comparison with betadiketones (SBBM) is also advantageous, these having an initially lighter colour but which decays rapidly to result in a worse long-term stability.

2-phenylindole has a better colour only a few minutes before burning.

The plate out was found low for all compounds (1-2).

EXAMPLE 6

Static stability was evaluated at 190 °C on the formulation for rigid pigmented articles (No. 2) using 0.3 PHR of organic stabilizer. the YI values are given.

| Time (minutes) | 1 | 2 | 3 | 4 | 5 |
|---|---|---|---|---|---|
| without stabilizer | 65.9 | 69 | 71.3 | 74.1 | 74.4 |
| DHPL (a) | 44.2 | 50 | 59.4 | 67.7 | 80 |
| BGD | 37.3 | 43.3 | 43.4 | 43.4 | 46.4 |
| TGD | 40.4 | 40.6 | 41.3 | 39.1 | 52.9 |
| TGM | 29.3 | 30.5 | 29.7 | 27.7 | 30.7 |

(a) used as reference

The compounds of the invention are excellent thermal stabilizers and are better than the structurally analogous one (a) normally used.

EXAMPLE 7

Dynamic stability was evaluated at 175 °C on a formulation for rigid transparent articles based on polyvinyl chloride-polyvinyl acetate copolymer in mixture with PVC (No. 1D) using 0.3 PHR of organic stabilizer. The YI values are given.

| Time (minutes) | 5 | 10 | 15 | 20 |
|---|---|---|---|---|
| without stabilizer | 24 | 31.1 | 50.2 | 87.7 |
| DHPL (a) | 10.9 | 12.7 | 35.6 | 85.1 |
| BGD | 9.8 | 11.9 | 24.3 | 77.2 |

(a) used as reference

The excellent thermal stability of the compounds of the invention can be seen, this being substantially better than that of currently used products.

EXAMPLE 8

Dynamic stability was evaluated at 180 °C on the formulation for plasticised articles (No. 3A) using 0.15 PHR of organic stabilizer. The YI values are given.

| Time (minutes) | 5 | 10 | 15 | 20 | 25 | 30 |
|---|---|---|---|---|---|---|
| without stabilizer | 10.5 | 14.4 | 18.7 | 19.8 | 17.7 | 28 |
| TGD | 9.1 | 8.4 | 7.7 | 8.2 | 10.1 | 17 |
| BGD | 8.5 | 7.9 | 7.6 | 8.1 | 11.4 | 16.5 |
| TGM | 10.5 | 9.3 | 8.2 | 8.0 | 9.7 | 16.1 |

Excellent thermal stability can be noted, with a particularly low colour until burning.

EXAMPLE 9

Dynamic stability was evaluated at 180°C for BGD with different PHR values on a formulation for rigid transparent articles (No. 1B).

| Time (minutes) | 3 | 6 | 9 | 12 | 15 | 18 | 21 |
|---|---|---|---|---|---|---|---|
| without stab. | 40 | 54.2 | 62.2 | 71.3 | 80.1 | 97.1 | 111.8 |
| 0.05 PHR | 23.1 | 28.7 | 30.6 | 36 | 58.5 | 88.2 | 111.2 |
| 0.15 PHR | 20.7 | 23.6 | 24.4 | 25.7 | 47.5 | 84.7 | 116.2 |
| 0.30 PHR | 17.1 | 18.2 | 18.9 | 19.6 | 22.5 | 38.5 | 86.7 |
| 0.50 PHR | 16.2 | 17.1 | 18.3 | 15.5 | 24.5 | 40.2 | 94.4 |

An excellent stabilization efficiency can be noted over a wide percentage incorporation range of the compounds according to the invention. In particular, the efficiency is already high at very small incorporation quantities and tends to improve as the amount incorporated increases until it reaches an optimum value at 0.3 PHR (compare YI at 15 and 18 minutes).

EXAMPLE 10

Dynamic stability was evaluated at 180°C on a formulation for rigid transparent articles (No. 1C) using 0.15 PHR of organic stabilizer (BGD) at different ratios of calcium to zinc stearate as primary stabilizer.

| Time (minutes) | 3 | 6 | 9 | 12 | 15 | 18 | 21 |
|---|---|---|---|---|---|---|---|
| Zinc stearate | | | | | | | |
| 0.1 PHR | 23.9 | 32 | 45.9 | 78 | 138.7 | - | - |
| 0.2 | 20.7 | 23.6 | 24.4 | 25.5 | 47.5 | 92.2 | 116.2 |
| 0.3 | 17.8 | 19.7 | 20.1 | 20.6 | 24.4 | 38.5 | 68.2 |

A sharp improvement in colour is noted together with a long-term thermal stability increase as the quantity of zinc stearate approaches that of calcium stearate (0.36 PHR).

EXAMPLE 11

Dynamic stability was evaluated at 180°C on a formulation for plasticised articles (No. 3B, primary

stabilizers Ba/Zn) using 0.3 PHR of organic stabilizer.

| Time (minutes) | 5 | 10 | 15 | 20 | 25 | 30 | 35 | 40 | 45 |
|---|---|---|---|---|---|---|---|---|---|
| without stab. | 19.2 | 27.6 | 31.2 | 30.6 | 26.9 | 24.5 | 21.7 | 21.9 | 61 |
| BGD | 16.1 | 22.3 | 24.1 | 23.7 | 21.4 | 21.1 | 19.1 | 23.9 | 69.5 |
| TGD | 16.1 | 21.6 | 24.0 | 23.0 | 20.6 | 19.1 | 18.6 | 53.5 | - |
| TGM | 15.7 | 21.7 | 22.6 | 21.3 | 19.3 | 18.1 | 17.3 | 23.4 | 68.2 |

The compounds of the invention contribute towards maintaining the colour of the stabilized masses within substantially narrower limits when subjected to temperature and mechanical stress. The plate out is very good and is low for all compounds (1-2).

EXAMPLE 12

The lubrication evaluation test was carried out at 190 °C on a mix for rigid transparent articles comprising S-PVC (K 58) 100 parts, MBS 6 parts, epoxidised soya oil 1 part, calcium stearate 0.2 parts and zinc stearate 0.1 part, using 0.5 PHR of organic stabilizer.

The time (in minutes) taken for the mix to stick to the mixer rollers when operating with a friction ratio of 1:1.25 is given.

| Organic stabilizer | - | TGD | BGD | TGM |
|---|---|---|---|---|
| Time | 21 min | 23 min | 22 min 30 sec | 22 min |

It can be noted that on using the compounds of the invention there are no negative effects on the mixture lubrication, and in fact the effects are slightly positive, the compounds with long-chain substituents (TGD and BGD) being slightly better than those with short-chain substituents (TGM).

EXAMPLE 13

The transparency test was carried out at 190 °C on a mix for rigid transparent articles (No. 1B) using 0.3 PHR of organic stabilizer. The contrast ratios are given.

| Stabilizer | - | TGD | BGD | TGM |
|---|---|---|---|---|
| Contrast ratio | 0.2707 | 0.2595 | 0.2604 | 0.2640 |

A substantial positive effect on the article transparency can be noted when using the compounds of the invention, this effect being slightly better for compounds with long-chain substituents.

The following examples relate to the application of compounds of formula (I) in association with compounds of formula (II).

EXAMPLE 14

Dynamic stability was evaluated at 180 °C on a formulation for rigid transparent articles (No. 1A) using the indicated quantities of organic stabilizer. The YI values are given.

| Stabilizer | | | | Time (minutes) | | | | |
|---|---|---|---|---|---|---|---|---|
| | | | | 5 | 10 | 15 | 20 | 25 |
| 1 | TGD | 0.3 | PHR | 16.8 | 18 | 19 | 26.2 | 61 |
| 2 | SBBM | 0.3 | PHR (a) | 10.2 | 15.6 | 29.8 | 52.6 | 93.7 |
| 3 | TGD | 0.15 | PHR | 8 | 11.1 | 21.6 | 50 | 92 |
| | SBBM | 0.15 | PHR | | | | | |
| 4 | BSM | 0.3 | PHR (b) | 9.8 | 14.2 | 23.6 | 43.2 | 79 |
| 5 | TGD | 0.15 | PHR | 8.8 | 11.3 | 18.4 | 36.3 | 71.2 |
| | BSM | 0.15 | PHR | | | | | |
| 6 | TGD | 0.15 | PHR | 9.7 | 11.5 | 17.9 | 40.6 | 92 |
| | SBBM | 0.15 | PHR | | | | | |
| 7 | SN | 0.8 | PHR (c) | 10.1 | 15.7 | 28.6 | 43.1 | 57.1 |

(a) Sebacoyl bisbenzoylmethane

(b) Benzoylstearoylmethane

(c) Tin octyl isooctylthioglycolate derivative (IRGASTAB 17MOKS) used as reference.

Clear synergism can be noted between the TGD and the sebacoyl bisbenzoylmethane (SBBM) or benzoylstearoylmethane (BSM). Even with small SBBM quantities (mixture 6) a very high efficiency is obtained, exceeding that of currently used tin compounds.

EXAMPLE 15

Dynamic stability was evaluated at 180° C on a formulation for rigid transparent articles (No. 1B) using 0.3 PHR of single or mixed organic stabilizers (0.15 + 0.15 PHR of synergic compounds in mixture). The YI values are given.

| Time (minutes) | 5 | 10 | 15 | 20 | 25 |
|---|---|---|---|---|---|
| BGD | 17.7 | 19.3 | 21.5 | 28.9 | 60 |
| TGD | 17.5 | 19.1 | 20.1 | 27.3 | 63 |
| BSM | 11.7 | 16.4 | 24.5 | 41.3 | 73.4 |
| SBBM | 10.1 | 13.2 | 22.9 | 33.4 | 68.9 |
| BGD + BSM | 11.0 | 15.2 | 20.3 | 30 | 66 |
| BGD + SBBM | 10 | 12.2 | 16.9 | 30.2 | 68 |
| TGD + BSM | 11.2 | 15.1 | 19.5 | 33 | 68.7 |
| TGD + SBBM | 9.7 | 12.2 | 16.9 | 25.9 | 57.3 |

Synergism can be noted between the compounds of the invention and the betadiketones particularly in maintaining the colour low until burning.

EXAMPLE 16

Thermal stability at 190 °C was evaluated on a formulation for rigid pigmented articles (No. 2) using 0.15 PHR of each organic stabilizer (total 0.3 PHR). The YI values are given.

| Time (minutes) | 1 | 2 | 3 | 4 | 5 |
|---|---|---|---|---|---|
| without stabilizer | 65.9 | 69 | 71.3 | 74.1 | 74.4 |
| TGD + SBBM | 17.2 | 20.4 | 24.6 | 33.6 | 75.4 |
| BGD + SBBM | 18.4 | 18.7 | 23 | 26 | 35.1 |
| TGM + SBBM | 16.7 | 18.5 | 18.2 | 20.7 | 29.6 |

The very high efficiency of the synergic compositions is noted compared with the PVC mass when stabilized only with primary stabilizers.

EXAMPLE 17

Dynamic stability at 180 °C was evaluated on a PVC/PVA-based formulation for rigid transparent articles (No.1D) using 0.15 PHR of each organic stabilizer (0.3 PHR in total). The YI values are given.

| Time (minutes) | 5 | 10 | 15 | 20 |
|---|---|---|---|---|
| without stabilizer | 24 | 31.1 | 50.2 | 87.7 |
| TGD + SBBM | 7.2 | 9.4 | 23.4 | 65.9 |
| BGD + SBBM | 7.5 | 11 | 31.7 | 83.6 |
| TGM + SBBM | 9.8 | 11 | 30 | 88.6 |

EXAMPLE 18

The test conducted in Example 17 was repeated on a formulation for plasticised transparent articles (No. 3). The YI values are given.

| Time (minutes) | 5 | 10 | 15 | 20 | 25 | 30 |
|---|---|---|---|---|---|---|
| without stabilizer | 10.5 | 14.4 | 18.7 | 19.8 | 17.7 | 28 |
| TGD + SBBM | 7.1 | 7.2 | 7.3 | 8.5 | 13 | 20.3 |
| BGD + SBBM | 7.3 | 7.1 | 7.2 | 8.7 | 13 | 19.5 |
| TGM + SBBM | 8.3 | 7.6 | 7.7 | 8.3 | 12.1 | 18.7 |

The results of Examples 17 and 18 show the high efficiency of the compositions based on the compounds according to the invention in association with betadiketones (in particular bisbetadiketones such as sebacoyl bisbenzoylmethane), both on rigid and on plasticised formulations.

**Claims**

**Claims for the following Contracting States : AT, BE, CH, DE, FR, GB, GR, IT, LI, LU, NL, SE**

**1.** Compounds of formula (I)

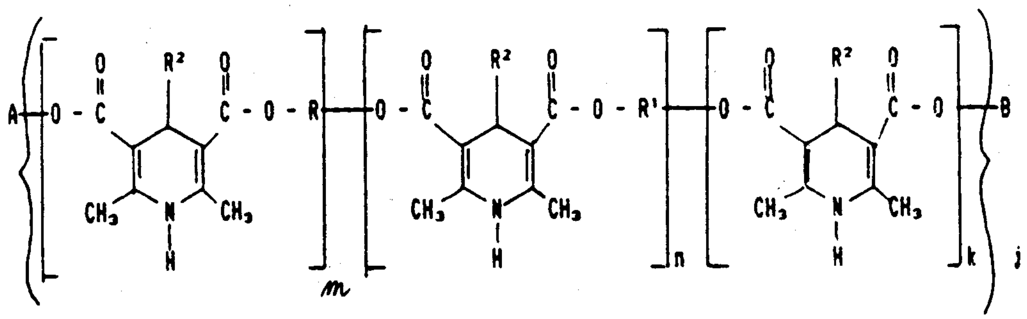

in which:
A represents a linear or branched alkyl of 1-22 C atoms, possibly substituted with one or more groups chosen from alkoxy, alkylthio, hydroxyl possibly esterified with acrylic or methacrylic acid, halogen, aryl; phenyl or aryl of carbocyclic or heterocyclic type possibly substituted with one or more alkyl, alkoxy or halogen groups; alkenyl of 3-10 C atoms; $CH_3COCH_2$-COO-R-; $CH_3COCH_2COO$-R'; $CH_3$-C-($NH_2$)=CH-COOR-; $CH_3$-C($NH_2$)=CHCOO-R' -; in which the amino group can carry one or more substituents of alkyl, hydroxyalkyl or alkoxyalkyl type or a cyclic substituent of polyalkylene or oxapolyalkylene type, or methylene or a linear or branched alkenyl of 2-22 C atoms;
B can assume the same meaning as A, or can represent a trivalent or polyvalent residue consisting of a linear or branched carbon atom chain possibly carrying substitutions of alkoxy, thioalkoxy, aryl, carboxyl or hydroxyl type;
m, n are whole numbers from 0 to 20, the sun of which is other than 0;
k can be 0 or 1;
j is a whole number from 1 to 6;
j (k+m+n) is a whole number greater than 1;
R and R' each independently represent a methylene or phenylene group, or one alkylene group of the type:

$$--(--C_pH_{2p}---X---)_t---C_pH_{2p}--$$

in which p is a whole number from 2 to 18, t is a whole number from 0 to 10 and X can be oxygen or sulphur, said alkylene group possibly carrying substitutions of alkoxy or thioalkoxy, aryl, carboxyl or hydroxyl type; or R and R' represent a direct bond with B only if k is 0 and j is greater than 1:
$R^2$ represents hydrogen or a monovalent residue of linear or branched alkyl, alkoxycarbonyl or aryl type possibly substituted with one or more alkyl, alkoxy, halogen or $NO_2$ groups.

**2.** Compounds of formula (I) as claimed in claim 1, characterised in that the sequence of units which are repeated m and n times, where m+n is greater than or equal to 3, can be of alternating, block or random type.

**3.** Compounds of formula (I) as claimed in claim 1, characterised in that:
A, B, which can be the same or different, each independently represent a linear or branched $C_1$-$C_{18}$ alkyl group;
m, k, j = 1;
n = 0;
R represents a -$(CH_2)_2$, -$(CH_2)_4$- or -$(CH_2)_2$-S-$(CH_2)_2$- group;
$R^2$ represents hydrogen.

**4.** A process for preparing compounds of formula (I) as defined in claim 1 characterised by condensing esters of betaminocrotonic acid with themselves, or with esters of acetoacetic acid, and with aldehyde compounds of $R^2$CHO type in which $R^2$ has the meaning defined in claim 1. thereby the molecular weight of said compounds of formula (I) is regulated by introducing an adequate predetermined quantity of monofunctional acetoacetate of formula $CH_3$-CO-$CH_2$-COO-X (V), or of betaaminocrotonate

of formula $H_2N-C(CH_3)=CH-COOX$ (VII), wherein $X=-CH_3$, $-C_2H_5$ or $-C_{12}H_{25}$.

**5.** A process as claimed in claim 4, characterised in that said esters of betaaminocrotonic acid are obtained in situ by reacting acetoacetic esters with ammonia, and are not isolated.

**6.** A process as claimed in claim 4, characterised in that said condensation is conducted in a medium consisting of a low-boiling alcoholic solvent, preferably isopropyl alcohol.

**7.** A process as claimed in claim 4, characterised in that said condensation is conducted by heating to a temperature of between 50 °C and the reflux temperature for a time of between 1 and 20 hours.

**8.** The use of compounds of formula (I) as defined in claims 1 to 3, as thermal stabilizers for synthetic polymers.

**9.** The use of compounds of formula (I) as defined in claims 1 to 3, as thermal stabilizers for polyvinylchloride-based masses.

**10.** Vinylchloride polymer or copolymer-based compositions containing usual additives, processing auxiliaries and primary stabilizers, comprising as thermal stabilizer at least one compound of formula (I) as defined in claims 1 to 3 to the extent of between 0.01% and 3% of the polymer by weight.

**11.** Compositions as claimed in claim 10, wherein the primary stabilizers include Ca/Zn or Mg/Zn salts of carboxylic acids, organotin mercaptides or carboxylates and their mixtures.

**12.** Compositions as claimed in claim 10, wherein the additives include betadiketone compounds of formula (II):

$R^3$-CO-$CH_2$-CO-$R^4$ (II)

in which:
$R^3$ represents linear or branched $C_1$-$C_{18}$ alkyl, or an aromatic phenyl ring possibly substituted with one or more alkyl, alkoxy or halogen groups;
$R^4$ has the same meaning as $R^3$, independently of $R^3$, or represents a covalent bond, $C_1$-$C_{18}$ alkylene, polyvalent $C_1$-$C_{18}$ hydrocarbon, or $-(C_pH_{2p}-X)_t-C_pH_{2p}-$ in which p, t, X have the meanings already given for formula (I), and directly linked to another -CO-$CH_2$-CO-$R^3$ group.

**13.** Compositions as claimed in claim 12, wherein the ratio of compounds of formula (I) to be betadiketone compounds of formula (II) is between 1:10 and 10:1.

**Claims for the following Contracting State : ES**

**1.** A process for preparing compounds of formula (I)

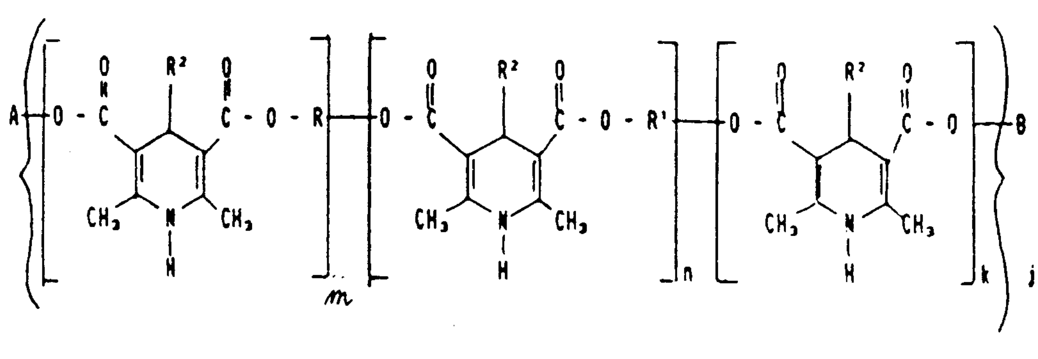

in which:
A represents a linear or branched alkyl of 1-22 C atoms, possibly substituted with one or more groups chosen from alkoxy, alkylthio, hydroxyl possibly esterified with acrylic or methacrylic acid, halogen,

aryl; phenyl or aryl of carbocyclic or heterocyclic type possibly substituted with one or more alkyl, alkoxy or halogen groups; alkenyl of 3-10 C atoms; $CH_3COCH_2$-COO-R-; $CH_3COCH_2COO$-$R^1$; $CH_3$-C $(NH_2)$=CH-COOR-; $CH_3$-C $(NH_2)$=CHCOO-$R^1$-; in which the amino group can carry one or more substituents of alkyl, hydroxyalkyl or alkoxyalkyl type or a cyclic substituent of polyalkylene or oxapolyalkylene type, or methylene or a linear or branched alkenyl of 2-22 C atoms;

B can assume the same meaning as A, or can represent a trivalent or polyvalent residue consisting of a linear or branched carbon atom chain possibly carrying substitutions of alkoxy, thioalkoxy, aryl, carboxyl or hydroxyl type;

m, n are whole numbers from 0 to 20, the sum of which is other than 0;

k can be 0 or 1;

j is a whole number from 1 to 6;

j (k + m + n) is a whole number greater than 1;

R and $R^1$ each independently represent a methylene or phenylene group, or one alkylene group of the type:

$$--(-- C_pH_{2p} ---X ---)_t--- C_p H_{2p} --$$

in which p is a whole number from 2 to 18, t is a whole number from 0 to 10 and X can be oxygen or sulphur, said alkylene group possibly carrying substitutions of alkoxy or thioalkoxy, aryl, carboxyl or hydroxyl type; or R and $R^1$ represent a direct bond with B only if k is 0 and j is greater than 1:

$R^2$ represents hydrogen or a monovalent residue of linear or branched alkyl, alkoxycarbonyl or aryl type possibly substituted with one or more alkyl, alkoxy, halogen or $NO_2$ groups, characterised by condensing esters of betaminocrotonic acid with themselves, or with esters of acetoacetic acid, and with aldehyde compounds of $R^2CHO$ type in which $R^2$ has the meaning defined above thereby the molecular weight of said compounds of formula (I) is regulated by introducing an adequate predetermined quantity of monofunctional acetoacetate of formula $CH_3$-CO-$CH_2$-COO-X (V), or of betaaminocrotonate of formula $H_2N$-$C(CH_3)$=CH-COOX (VII), wherein X = -$CH_3$, -$C_2H_5$ or -$C_{12}H_{25}$.

**2.** A process as claimed in claim 1, characterised in that said esters of betaaminocrotonic acid are obtained in situ by reacting acetoacetic esters with ammonia, and are not isolated.

**3.** A process as claimed in claim 1, characterised in that said condensation is conducted in a medium consisting of a low-boiling alcoholic solvent, preferably isopropyl alcohol.

**4.** A process as claimed in claim 1, characterised in that said condensation is conducted by heating to a temperature of between 50° C and the reflux temperature for a time of between 1 and 20 hours.

**5.** The use of compounds of formula (I) prepared by the process as defined in claim 1, as thermal stabilisers for synthetic polymers.

**6.** The use of compounds of formula (I) prepared by the process as defined in claim 1, as thermal stabilisers for polyvinylchloride-based masses.

**Patentansprüche**

**Patentansprüche für folgende Vertragsstaaten : AT, BE, CH, DE, FR, GB, GR, IT, LI, LU, NL, SE**

**1.** Verbindungen der Formel (I)

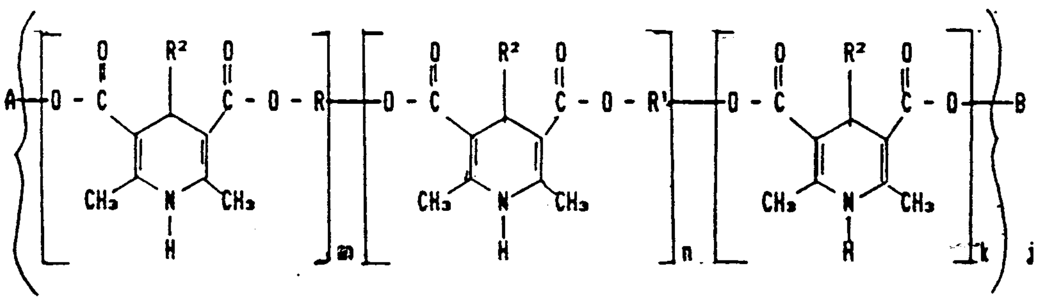

worin A lineares oder verzweigtes Alkyl mit 1 bis 22 C-Atomen, gegebenenfalls substituiert mit einer oder mehreren Gruppen ausgewählt aus Alkoxy, Alkylthio, Hydroxyl gegebenenfalls verestert mit Acryl- oder Methacrylsäure, Halogen, Aryl; Phenyl oder Aryl vom carbocyclischen oder heterocyclischen Typ gegebenenfalls substituiert mit einer oder mehreren Alkyl-, Alkoxy- oder Halogengruppen; Alkenyl mit 3 bis 10 C-Atomen; $CH_3COCH_2$-COO-R-; $CH_3COCH_2COO$-$R^1$; $CH_3$-$C(NH_2)$ = CH-COOR-; $CH_3$-$C(NH_2)$-= CHCOO-$R^1$-; worin die Aminogruppe einen oder mehrere Substituenten vom Alkyl-, Hydroxyalkyl- oder Alkoxyalkyltyp oder einen cyclischen Substituenten vom Polyalkylen- oder Oxapolyalkylentyp oder Methylen oder ein lineares oder verzweigtes Alkenyl mit 2 bis 22 C-Atomen tragen kann;

B die gleiche Bedeutung wie A haben kann oder einen drei-oder mehrwertigen Rest bestehend aus einer linearen oder verzweigten Kohlenstoffatomkette darstellen kann, die Substitutionen vom Alkoxy-, Thioalkoxy-, Aryl-, Carboxyl- oder Hydroxyltyp tragen kann;

m und n ganze Zahlen von Null bis 20 sind, deren Summe von Null verschieden ist;

k Null oder 1 sein kann;

j eine ganze Zahl von 1 bis 6 ist;

j (k + m + n) eine ganze Zahl größer als 1 ist;

R und $R^1$ jeweils unabhängig eine Methylen- oder Phenylengruppe oder eine Alkylengruppe vom Typ

$$-(-C_pH_{2p} - X - )_t - C_pH_{2p}-$$

bedeuten, wobei p eine ganze Zahl von 2 bis 18 ist, t eine ganze Zahl von Null bis 10 ist und X Sauerstoff oder Schwefel sein kann, wobei die Alkylengruppe gegebenenfalls Substitutionen vom Alkoxy- oder Thioalkoxy-, Aryl-, Carboxyl- oder Hydroxyltyp trägt; oder R und $R^1$ eine Direktbindung mit B darstellen, aber nur wenn k Null ist und j größer als 1 ist;

$R^2$ Wasserstoff oder einen einwertigen Rest vom linearen oder verzweigten Alkyl-, Alkoxycarbonyl- oder Aryltyp gegebenenfalls substituiert mit einer oder mehreren Alkyl-, Alkoxy-, Halogen-oder $NO_2$-Gruppen darstellt.

2. Verbindungen der Formel (I) nach Anspruch 1, dadurch gekennzeichnet, daß die Sequenz von Einheiten, die m- und n-fach wiederholt sind, wobei m + n größer als oder gleich 3 ist, vom alternierenden, Block- oder regellosen Typ sein kann.

3. Verbindungen der Formel (I) nach Anspruch 1, dadurch gekennzeichnet, daß A und B, die gleich oder verschieden sein können, jeweils unabhängig eine lineare oder verzweigte $C_1$-$C_{18}$-Alkylgruppe bedeuten,

m, k, j = 1,

n = Null,

R eine $-(CH_2)_2-$, $-(CH_2)_4-$ oder $-(CH_2)_2-S-(CH_2)_2-$Gruppe ist und

$R^2$ Wasserstoff darstellt.

4. Verfahren zum Herstellen von Verbindungen der Formel (I), wie in Anspruch 1 definiert, gekennzeichnet durch Kondensieren von Estern von $\beta$-Aminocrotonsäure mit sich selbst oder mit Estern von Acetoessigsäure und mit Aldehydverbindungen der Formel $R^2CHO$, worin $R^2$ die in Anspruch 1 angegebene Bedeutung hat, wobei das Molgewicht der Verbindungen der Formel (I) reguliert wird, indem eine adäquate vorherbestimmte Menge von monofunktionellem Acetoacetat der Formel $CH_3CO$-$CH_2$-COO-X (V) oder von $\beta$-Aminocrotonat der Formel $H_2N$-$C(CH_3)$ = CH-COOX (VII), worin X = $-CH_3$, $-C_2H_5$ oder

$-C_{12}H_{25}$, eingeführt wird.

5. Verfahren nach Anspruch 4, dadurch gekennzeichnet, daß die Ester von $\beta$-Aminocrotonsäure in situ durch Umsetzen von Acetoessigsäureestern mit Ammoniak erhalten und nicht isoliert werden.

6. Verfahren nach Anspruch 4, dadurch gekennzeichnet, daß die Kondensation in einem Medium bestehend aus einem niedrigsiedenden alkoholischen Lösungsmittel, vorzugsweise Isopropylalkohol, durchgeführt wird.

7. Verfahren nach Anspruch 4, dadurch gekennzeichnet, daß die Kondensation durch Erhitzen auf eine Temperatur von 50°C bis zur Rückflußtemperatur während einer Zeit von 1 bis 20 Stunden durchgeführt wird.

8. Verwendung von Verbindungen der Formel (I), wie in den Ansprüchen 1 bis 3 definiert, als Wärmestabilisatoren für synthetische Polymere.

9. Verwendung von Verbindungen der Formel (I), wie in den Ansprüchen 1 bis 3 definiert, als Wärmestabilisatoren für Massen auf Polyvinylchloridbasis.

10. Zusammensetzungen auf Vinylchloridpolymer- oder -copolymerbasis enthaltend übliche Additive, Verarbeitungshilfsmittel und primäre Stabilisatoren, die als Wärmestabilisator mindestens eine Verbindung der Formel (I), wie in den Ansprüchen 1 bis 3 definiert, in einem Anteil von 0,01 bis 3 Gew.% des Polymers aufweisen.

11. Zusammensetzungen nach Anspruch 10, in welchen die primären Stabilisatoren Ca/Zn- oder Mg/Zn-Salze von Carbonsäuren, Organozinnmercaptide oder -carboxylate und deren Mischungen sind.

12. Zusammensetzungen nach Anspruch 10, in denen die Additive $\beta$-Diketonverbindungen der Formel (II)

$R^3$-CO-$CH_2$-CO-$R^4$ (II),

worin $R^3$ lineares oder verzweigtes $C_1$-$C_{18}$ -Alkyl oder einen aromatischen Phenylring gegebenenfalls substituiert mit einer oder mehreren Alkyl-, Alkoxy- oder Halogengruppen bedeutet, $R^4$ die gleiche Bedeutung wie $R^3$ unabhängig von $R^3$ hat oder eine kovalente Bindung, $C_1$-$C_{18}$-Alkylen, einen mehrwertigen $C_1$-$C_{18}$-Kohlenwasserstoff oder $-(C_pH_{2p}-X)_t-C_pH_{2p}$, worin p, t und X die für Formel (I) angegebenen Bedeutungen haben, darstellt und direkt an eine andere -CO-$CH_2$-CO-$R^3$-Gruppe gebunden ist.

13. Zusammensetzungen nach Anspruch 12, in welchen das Verhältnis der Verbindungen der Formel (I) zu den $\beta$-Diketonverbindungen der Formel (II) 1 : 10 bis 10 : 1 beträgt.

**Patentansprüche für folgenden Vertragsstaat : ES**

1. Verfahren zum Herstellen von Verbindungen der Formel (I)

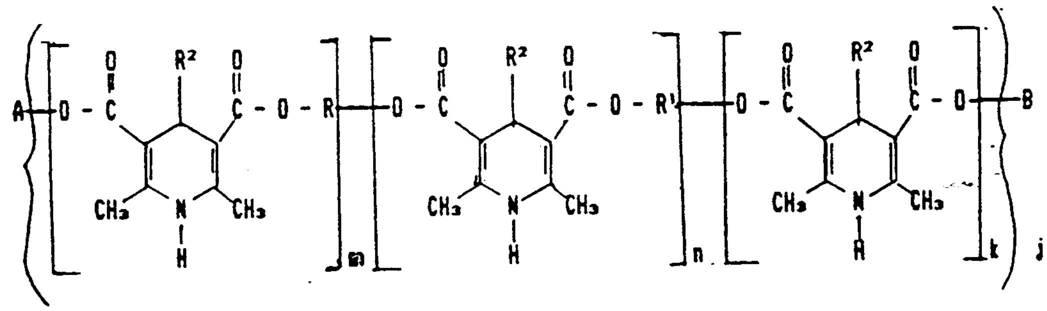

worin A lineares oder verzweigtes Alkyl mit 1 bis 22 C-Atomen, gegebenenfalls substituiert mit einer

oder mehreren Gruppen ausgewählt aus Alkoxy, Alkylthio, Hydroxyl gegebenenfalls verestert mit Acryl- oder Methacrylsäure, Halogen, Aryl; Phenyl oder Aryl vom carbocyclischen oder heterocyclischen Typ gegebenenfalls substituiert mit einer oder mehreren Alkyl-, Alkoxy- oder Halogengruppen; Alkenyl mit 3 bis 10 C-Atomen; $CH_3COCH_2$-COO-R-; $CH_3COCH_2COO$-$R^1$; $CH_3$-C($NH_2$)=CH-COOR-; $CH_3$-C($NH_2$)-=CHCOO-$R^1$-; worin die Aminogruppe einen oder mehrere Substituenten vom Alkyl-, Hydroxyalkyl- oder Alkoxyalkyltyp oder einen cyclischen-Substituenten vom Polyalkylen- oder Oxapolyalkylentyp oder Methylen oder ein lineares oder verzweigtes Alkenyl mit 2 bis 22 C-Atomen tragen kann;

B die gleiche Bedeutung wie A haben kann oder einen drei-oder mehrwertigen Rest bestehend aus einer linearen oder verzweigten Kohlenstoffatomkette darstellen kann, die Substitutionen vom Alkoxy-, Thioalkoxy-, Aryl-, Carboxyl- oder Hydroxyltyp tragen kann;

m und n ganze Zahlen von Null bis 20 sind, deren Summe von Null verschieden ist;

k Null oder 1 sein kann;

j eine ganze Zahl von 1 bis 6 ist;

j (k + m + n) eine ganze Zahl größer als 1 ist;

R und $R^1$ jeweils unabhängig eine Methylen- oder Phenylengruppe oder eine Alkylengruppe vom Typ

$$-(-C_pH_{2p} - X - )_t - C_pH_{2p}-$$

bedeuten, wobei p eine ganze Zahl von 2 bis 18 ist, t eine ganze Zahl von Null bis 10 ist und X Sauerstoff oder Schwefel sein kann, wobei die Alkylengruppe gegebenenfalls Substitutionen vom Alkoxy- oder Thioalkoxy-, Aryl-, Carboxyl- oder Hydroxyltyp trägt; oder R und $R^1$ eine Direktbindung mit B darstellen, aber nur wenn k Null ist und j größer als 1 ist;

$R^2$ Wasserstoff oder einen einwertigen Rest vom linearen oder verzweigten Alkyl-, Alkoxycarbonyl- oder Aryltyp gegebenenfalls substituiert mit einer oder mehreren Alkyl-, Alkoxy-, Halogen-oder $NO_2$-Gruppen darstellt, gekennzeichnet durch Kondensieren von Estern von β-Aminocrotonsäure mit sich selbst oder mit Estern von Acetoessigsäure und mit Aldehydverbindungen der Formel $R^2$CHO, worin $R^2$ die oben angegebene Bedeutung hat, wobei das Molgewicht der Verbindungen der Formel (I) reguliert wird, indem eine adäquate vorherbestimmte Menge von monofunktionellem Acetoacetat der Formel $CH_3CO$-$CH_2$-COO-X (V) oder von β-Aminocrotonat der Formel $H_2N$-C($CH_3$)=CH-COOX (VII), worin X = -$CH_3$, -$C_2H_5$ oder -$C_{12}H_{25}$, eingeführt wird.

**2.** Verfahren nach Anspruch 1, dadurch gekennzeichnet, daß die Ester von β-Aminocrotonsäure in situ durch Umsetzen von Acetoessigsäureestern mit Ammoniak erhalten und nicht isoliert werden.

**3.** Verfahren nach Anspruch 1, dadurch gekennzeichnet, daß die Kondensation in einem Medium bestehend aus einem niedrigsiedenden alkoholischen Lösungsmittel, vorzugsweise Isopropylalkohol, durchgeführt wird.

**4.** Verfahren nach Anspruch 1, dadurch gekennzeichnet, daß die Kondensation durch Erhitzen auf eine Temperatur von 50 °C bis zur Rückflußtemperatur während einer Zeit von 1 bis 20 Stunden durchgeführt wird.

**5.** Verwendung der Verbindungen der Formel (I), hergestellt durch das Verfahren nach Anspruch 1, als Wärmestabilisatoren für synthetische Polymere.

**6.** Verwendung der Verbindungen der Formel (I), hergestellt durch das Verfahren nach Anspruch 1, als Wärmestabilisatoren für Massen auf Polyvinylchloridbasis.

**Revendications**
**Revendications pour les Etats contractants suivants : AT, BE, CH, DE, FR, GB, GR, IT, LI, LU, NL, SE**

**1.** Composés de formule (I)

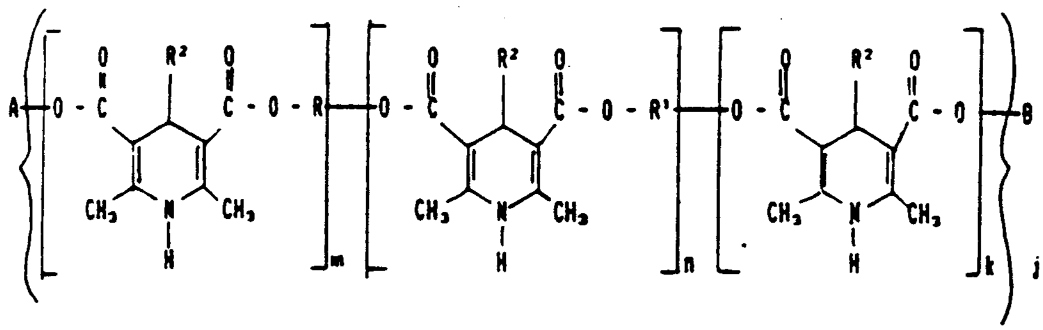

dans laquelle

A représente un alkyle linéaire ou ramifié contenant de 1 à 22 atomes de carbone, éventuellement substitués par un ou plusieurs groupes choisis parmi des alcoxy, alkylthio, hydroxyle, pouvant être esterifiés par l'acide acrylique ou méthacrylique, un halogène, un radical aryle; un radical phényle ou aryle de type carbocyclique ou hétérocyclique pouvant être substitué par un ou plusieurs groupes alkyle, alcoxy ou halogène; un alcényle ayant de 3 à 10 atomes de carbone; $CH_3COCH_2$-COO-R-; $CH_3COCH_2$-COO-$R^1$; $CH_3$-C($NH_2$) = CH-COOR-; $CH_3$-C($NH_2$) = CHCOO-$R^1$-; dans lesquels le groupe amino peut porter un ou plusieurs substituants de type alkyle, hydroxyalkyle ou alcoxyalkyle ou un substituant cyclique de type polyalkylène ou oxapolyalkylène, ou méthylène ou un alcényle linéaire ou ramifié ayant de 2 à 22 atomes de carbone;

B peut avoir la même signification que A, ou peut représenter un reste trivalent ou polyvalent constitué par une chaîne de carbone linéaire ou ramifiée pouvant porter des substitutions de type alcoxy, thioalcoxy, aryle, carboxyle ou hydroxyle;

m,n sont des nombres entiers pouvant aller de 0 à 20, dont la somme est différente de 0;

k peut être 0 ou 1;

j est un nombre entier pouvant aller de 1 à 6;

j (k + m + n) est un nombre entier supérieur à 1;

R et $R^1$ représentent, chacun indépendamment, un groupe éthylène ou un groupe phénylène, ou un groupe de type alkylène :

$$--(-- C_pH_{2p} ---X ---)_t--- C_pH_{2p} --$$

dans lequel p est un nombre entier pouvant aller de 2 à 18, t est un nombre entier pouvant aller de 0 à 10 et X peut être l'oxygène ou le soufre, ledit groupe alkylène pouvant porter des substitutions de type alcoxy ou thioalcoxy, aryle, carboxyle ou hydroxyle; ou R et $R^1$ représentent une liaison directe avec B seulement si k = 0 et j supérieur à 1;

$R^2$ représente l'hydrogène ou un reste monovalent de type alkyle, alcoxycarbonyle ou aryle linéaire ou ramifié, pouvant être substitué par un ou plusieurs groupes alkyle, alcoxy, halogène ou $NO_2$.

**2.** Composés de formule (I), tels que revendiqués dans la revendication 1, caractérisés en ce que la séquence de motifs qui sont répétés m et n fois, où m + n est supérieur ou égal à 3, peut être de type alterné, séquencé ou stochastique.

**3.** Composés de formule (I) tels que revendiqués dans la revendication 1, caractérisés en ce que :

A, B, qui peuvent être identiques ou différents, représentent chacun indépendamment un groupe alkyle linéaire ou ramifié en $C_1$ à $C_{18}$ ;

m, k, j = 1;

n = 0;

R représente un groupe $-(CH_2)_2-$, un groupe $-(CH_2)_4$ - ou un groupe $-(CH_2)_2-S-(CH_2)_2-$;

$R^2$ représente l'hydrogène.

**4.** Procédé de préparation de composés de formule (I), tels que définis dans la revendication 1, caractérisé par le fait que l'on condense des esters de l'acide bétaaminocrotonique avec eux-mêmes, ou avec des esters de l'acide acétoacétique, et avec des composés aldéhydes de type $R^2CHO$ dans lesquels $R^2$ a la signification définie dans la revendication 1, grâce à quoi la masse moléculaire desdits

composés de formule (I) se trouve régulée par l'introduction d'une quantité adéquate prédéterminée d'acétoacétate monofonctionnel de formule $CH_3$-CO-$CH_2$-COO-X (V) ou de bétaaminocrotonate de formule $H_2$N-C($CH_3$) = CH-COOX (VII), dans laquelle X = -$CH_3$, -$C_2H_5$ ou $C_{12}H_{25}$ .

**5.** Procédé tel que revendiqué dans la revendication 4, caractérisé en ce que lesdits esters de l'acide bétaaminocrotonique sont obtenus in situ en faisant réagir des esters acétoacétiques avec l'ammoniac, et ne sont pas isolés.

**6.** Procédé tel que revendiqué dans la revendication 4, caractérisé en ce que ladite condensation est effectuée dans un milieu constitué par un solvant alcoolique à bas point de fusion, de préférence l'alcool isopropylique.

**7.** Procédé tel que revendiqué dans la revendication 4, caractérisé en ce que ladite condensation est effectuée par chauffage à une température comprise entre 50° C et la température de reflux, pendant une durée comprise entre 1 heure et 20 heures.

**8.** Utilisation de composés de formule (I) tels que définis dans les revendications 1 à 3, en tant que stabilisants thermiques pour des polymères synthétiques.

**9.** Utilisation de composés de formule (I) tels que définis dans les revendications 1 à 3, en tant que stabilisants thermiques pour des matières à base de chlorure de polyvinyle.

**10.** Polymère de chlorure de vinyle ou compostions à base de copolymère contenant des additifs usuels, des auxiliaires de traitement et des stabilisants primaires, comprenant à tire de stabilisant thermique au moins un composé de formule (I) tel que défini dans les revendications 1 à 3, à une teneur comprise entre 0,01 % et 3 % du polymère en poids.

**11.** Compositions telles que revendiquées dans la revendication 10, dans lesquelles les stabilisants primaires comprennent des sels Ca/Zn ou Mg/Zn d'acides carboxyliques, des mercaptides organostanneux ou des carboxylates et leurs mélanges.

**12.** Compositions telles que revendiquées dans la revendication 10, dans lesquelles les additifs comprennent des composés bétadicétoniques de formule (II) :

$R^3$-CO-$CH_2$-CO-$R^4$ (II)

dans laquelle
$R^3$ représente un alkyle en $C_1$ à $C_{18}$ linéaire ou ramifié ou un cycle phényle aromatique, pouvant être substitué avec un ou plusieurs groupes alkyle, alcoxy ou halogène;
$R^4$ a la même signification que $R^3$, indépendamment de $R^3$, ou représente une liaison covalente, un alkylène en $C_1$ à $C_{18}$, un hydrocarbure polyvalent en $C_1$ à $C_{18}$, ou -$(C_pH_{2p}$-X$)_t$-$C_pH_{2p}$- dans lequel p, t, X ont les significations déjà données pour la formule (), et directement lié à un autre groupe -CO-$CH_2$-CO-$R^3$.

**13.** Compositions telles que revendiquées dans la revendication 12, dans lesquelles le rapport des composés de formule (I) aux composés bétadicétoniques de formule (II) est compris entre 1 : 10 et 10 : 1.

**Revendications pour l'Etat contractant suivant : ES**

**1.** Procédé de préparation de composés de formule (I)

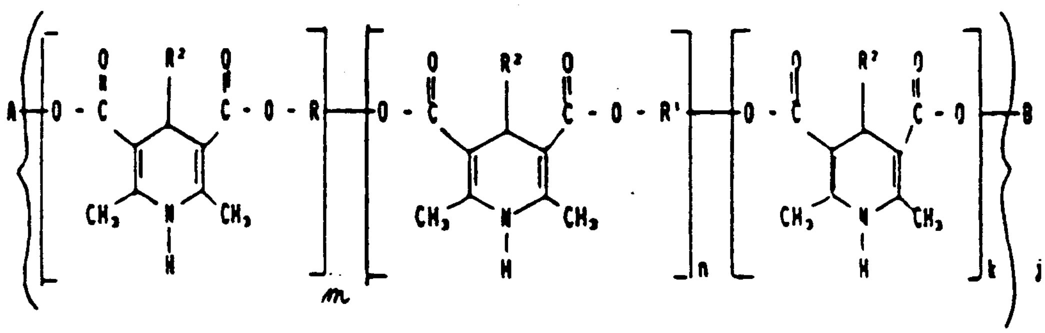

dans laquelle

A représente un alkyle linéaire ou ramifié contenant de I à 22 atomes de carbone, éventuellement substitués par un ou plusieurs groupes choisis parmi des alcoxy, alkylthio, hydroxyle, pouvant être esterifiés par l'acide acrylique ou méthacrylique, un halogène, un radical aryle; un radical phényle ou aryle de type carbocyclique ou hétérocyclique pouvant être substitué par un ou plusieurs groupes alkyle, alcoxy ou halogène; un alcényle ayant de 3 à 10 atomes de carbone; $CH_3COCH_2$-COO-R-; $CH_3COCH_2$-COO-$R^1$; $CH_3$-C($NH_2$) = CH-COOR-; $CH_3$-C($NH_2$) = CHCOO-$R^1$-; dans lesquels le groupe amino peut porter un ou plusieurs substituants de type alkyle, hydroxyalkyle ou alcoxyalkyle ou un substituant cyclique de type polyalkylène ou oxapolyalkylène, ou méthylène ou un alcényle linéaire ou ramifié ayant de 2 à 22 atomes de carbone;

B peut avoir la même signification que A, ou peut représenter un reste trivalent ou polyvalent constitué par une chaîne de carbone linéaire ou ramifiée pouvant porter des substitutions de type alcoxy, thioalcoxy, aryle, carboxyle ou hydroxyle;

m,n sont des nombres entiers pouvant aller de 0 à 20, dont la somme est différente de 0;

k peut être 0 ou 1;

j est un nombre entier pouvant aller de 1 à 6;

j (k + m + n) est un nombre entier supérieur à 1;

R et $R^1$ représentent, chacun indépendamment, un groupe éthylène ou un groupe phénylène, ou un groupe de type alkylène :

$$--(-- C_pH_{2p} ---X ---)_t--- C_pH_{2p} --$$

dans lequel p est un nombre entier pouvant aller de 2 à 18, t est un nombre entier pouvant aller de 0 à 10 et X peut être l'oxygène ou le soufre, ledit groupe alkylène pouvant porter des substitutions de type alcoxy ou thioalcoxy, aryle, carboxyle ou hydroxyle; ou R et $R^1$ représentent une liaison directe avec B seulement si k = 0 et j supérieur à 1;

$R^2$ représente l'hydrogène ou un reste monovalent de type alkyle, alcoxycarbonyle ou aryle linéaire ou ramifié, pouvant être substitué par un ou plusieurs groupes alkyle, alcoxy, halogène ou $NO_2$, caractérisé par le fait que l'on condense des esters de l'acide bétaaminocrotonique avec eux-mêmes, ou avec des esters de l'acide acétoacétique, et avec des composés aldéhydes de type $R^2$CHO dans lesquels $R^2$ a la signification définie ci-dessus, grâce à quoi la masse moléculaire desdits composés de formule (I) se trouve régulée par l'introduction d'une quantité adéquate prédéterminée d'acétoacétate monofonctionnel de formule $CH_3$-CO-$CH_2$-COO-X (V) ou de bétaaminocrotonate de formule $H_2$N-C-($CH_3$) = CH-COO-X (VII), dans laquelle X = -$CH_3$, $C_2H_5$, ou $C_{12}H_{25}$ .

**2.** Procédé tel que revendiqué dans la revendication 1, caractérisé en ce que lesdits esters de l'acide bétaaminocrotonique sont obtenus in situ, en faisant réagir des esters acétoacétiques avec l'ammoniac, et ne sont pas isolés.

**3.** Procédé tel que revendiqué dans la revendication 1, caractérisé en ce que ladite condensation est effectuée dans un milieu constitué par un solvant alcoolique a bas point de fusion, de préférence l'alcool isopropylique.

**4.** Procédé tel que revendiqué dans la revendication 1, caractérisé en ce que ladite condensation est effectuée par chauffage à une température comprise entre 50° C et la température de reflux, pendant

une durée comprise entre 1 heure et 20 heures.

**5.** Utilisation de composés de formule (I) préparés suivant le procédé tel que défini dans la revendication 1, en tant que stabilisants thermiques pour des polymères synthétiques.

**6.** Utilisation de composés de formule (I) préparés suivant le procédé tel que défini dans la revendication 1, en tant que stabilisants thermiques pour des matières à base de chlorure de polyvinyle.